(19) Europäisches Patentamt / European Patent Office / Office européen des brevets

(11) **EP 1 241 976 B1**

(12) **EUROPÄISCHE PATENTSCHRIFT**

(45) Veröffentlichungstag und Bekanntmachung des Hinweises auf die Patenterteilung:
**18.06.2008  Patentblatt 2008/25**

(51) Int Cl.:
*A61B 3/113* *(2006.01)*          *G06F 3/00* *(2006.01)*
*A61B 5/00* *(2006.01)*

(21) Anmeldenummer: **00987012.2**

(22) Anmeldetag: **17.10.2000**

(86) Internationale Anmeldenummer:
**PCT/DE2000/003728**

(87) Internationale Veröffentlichungsnummer:
**WO 2001/034021 (17.05.2001 Gazette 2001/20)**

(54) **VERFAHREN UND ANORDNUNG ZUM ERFASSEN VON MEHRDIMENSIONALEN AUGENBEWEGUNGEN**

METHOD AND ASSEMBLY FOR DETECTING MULTI-DIMENSIONAL EYE MOVEMENTS

PROCEDE ET AGENCEMENT POUR LA DETECTION DE MOUVEMENTS OCULAIRES MULTIDIMENSIONNELS

(84) Benannte Vertragsstaaten:
**AT BE CH CY DE DK ES FI FR GB GR IE IT LI LU MC NL PT SE**

(30) Priorität: **29.10.1999  DE 19954047**

(43) Veröffentlichungstag der Anmeldung:
**25.09.2002  Patentblatt 2002/39**

(73) Patentinhaber: **Chronos Vision GmbH**
**12247 Berlin (DE)**

(72) Erfinder: **CLARKE, Andrew, A.**
**14163 Berlin (DE)**

(74) Vertreter: **Willems, Volker**
**Rathausstrasse 28**
**82024 Taufkirchen (DE)**

(56) Entgegenhaltungen:
EP-A- 0 350 957          WO-A-99/23936
US-A- 4 848 340          US-A- 5 620 436
US-A- 5 632 742

• **PATENT ABSTRACTS OF JAPAN vol. 017, no. 171 (C-1044), 2. April 1993 (1993-04-02) & JP 04 329925 A (KOONAN:KK;OTHERS: 01), 18. November 1992 (1992-11-18) in der Anmeldung erwähnt**

EP 1 241 976 B1

**Beschreibung**

Die Erfindung betrifft ein Verfahren zum Erfassen von mehrdimensionalen Augenbewegungen, insbesondere horizontalen, vertikalen und torsionalen Augenbewegungen, bei dem das Auge mit diffusem Infrarotlicht beleuchtet wird und durch einen optoelektronischen Sensor Bildsequenzen des Auges erfasst werden, die einer dedizierten Rechenarchitektur zur Verarbeitung zugeführt werden, die die Koordinaten der Augenbewegung bestimmt und wahlweise die Bildsequenzen mit den ermittelten Koordinaten aufzeichnet und auf einem Monitor anzeigt.

Die Erfindung betrifft ferner eine Anordnung zum Erfassen von mehrdimensionalen Augenbewegungen, insbesondere horizontalen, vertikalen und torsionalen Augenbewegungen, mit einer diffusen IR-Lichtquelle zur Beleuchtung des Auges, einem optoelektronischen Sensor zur Erfassung von Bildsequenzen des Auges, und einer dedizierten Rechenarchitektur zur Bildverarbeitung für die Bestimmung der Augenbewegungen mittels künstlich auf der Bindehaut in einer vorgegebenen Geometrie aufgebrachten Markierungen.

Des weiteren wird eine Tinktur zur künstlichen Markierung der Bindehaut des Auges für die Erfassung von mehrdimensionalen Augenbewegungen beschrieben.

Die Messung von Augenbewegungen ist ein wesentliches Hilfsmittel für Wissenschaftler in den unterschiedlichsten Arbeitsgebieten, beispielsweise Physiologen, Psychologen, Ergonomen, Ophthalmologen und Neurologen. Dementsprechend steht die Entwicklung bzw. Verbesserung universell einsetzbarer hochauflösender Messtechniken stets im Mittelpunkt des allgemeinen Interesses.

Das menschliche Auge wird von drei mehr oder weniger rechtwinklig zueinander angeordneten Muskelpaaren positioniert bzw. bewegt. Dies erlaubt die Rotation des Auges um die drei orthogonalen Achsen des Auges im Kopf. Allgemein bezeichnet man diese Komponenten als horizontale, vertikale und torsionale Rotationskomponenten.

In der Vergangenheit wurden zunächst Methoden zur ein- bzw. zweidimensionalen Messung der Augenbewegungen, d. h. zur Messung der horizontalen und vertikalen Komponenten der Augenbewegungen, entwickelt. Darunter sind fotografische bzw. video-okulografische Ansätze zu finden, die auf einem sogenannten "Objekt-Tracking" im Bild basieren (CLARKE A. H., TEIWES W, SCHERER, H."Videooculography - an alternative method for measurement of three-dimensional eye movements", 1991, S. 431-443 in Schmidt R. Zambarbieri D (Eds) Oculomotor Control and Cognitive Processes Elsevier, Amsterdam).

Diese bekannten auf Bildverarbeitung basierenden Systeme gestatten die Messungen der horizontalen und vertikalen Augenposition mit Meßgenauigkeiten von 0,1 Grad über einen Bereich von +/- 20 Grad. Dabei wird vorausgesetzt, dass der Pupillenmittelpunkt einen festen Punkt auf dem Augenbulbus, und somit als akkurater Indikator der horizontalen und vertikalen Position des Auges im Kopf dient. Neuerdings wird diese Annahme in Frage gestellt (WYATT, H.J. (1995) "The form of the human pupil", Vision Res. 35, 14, 2021-2036). In diesem bekannten Stand der Technik wird festgestellt, dass der Schwerpunkt der Pupille bei Konstriktion bzw. Dilatation variiert.

Zur dreidimensionalen Messung der Augenbewegung, d.h. der horizontalen, vertikalen und torsionalen Rotationskomponenten, sind prinzipiell zwei Lösungsansätze bekannt.

So erlaubt die auf elektromagnetische Induktion basierende sklerale "Search-Coil"-Technik die genaueste Messung der Rotation des Auges um alle drei Achsen. Bei dieser im Jahre 1963 eingeführten Methode wird eine in einem kontaktlinsenähnlichen Annulus eingebettete Spule mit etwa 10 Windungen auf das zu messende Auge aufgesetzt, während sich der Kopf des Probanden innerhalb eines homogenen Magnetfeldes befindet. Nach dem elektromagnetischen Induktionsgesetz wird eine Spannung proportional zum Winkel zwischen der Spule und dem magnetischen Feld induziert. Diese Spannung gibt die Winkelposition des Auges im Raum wieder

Trotz ihrer hohen Auflösung hat die "Search-Coil-Technik" eine Reihe von Nachteilen. Der Proband muß eine Haftschale mit der eingebetteten Drahtspule auf dem Auge tragen. Dies bedeutet einen halb-invasiven Eingriff mit einigen Risiken für den Probanden. Außerdem verursachen die individuell verschiedenen Krümmungen des Auges bzw. der Kornea Probleme bei der Kalibrierung. Darüber hinaus kommt es immer wieder zum Wegrutschen der Haftschale, beispielsweise durch Blinzeln. Bedingt durch das Prinzip der elektromagnetischen Induktion erlaubt diese Meßtechnik eine Messung der Rotation, nicht aber der Translation des Auges. Hinzu kommt, dass der elektromagnetische Aufbau durch diverse metallische Gegenstände gestört werden kann. Schließlich sind die Kosten der verwendeten Haftschalen hoch. Die Lebensdauer der Haftschalen ist mit 1 bis 6 Messungen relativ gering.

Die Messung der dreidimensionalen Augenbewegungen mit Hilfe der Bildverarbeitungstechnik (Video-Okulographie) ist aus CLARKE A.H., TEIWES W., SCHERER H. ("Videooculography - an alternativ method for the registration of eye movements in three dimensions", 5th European Conference on Eye Movements, Pavia, 1993), CLARKE A.H. ("Image processing techniques for the measurement of eye movement", Ygge J, Lennerstrand G (Eds), Eye Movements in Reading, Elsevier, Oxford, NY pp 21-38, 1991), CLARKE A.H. ("Neuere Aspekte des vestibulookulären Reflexes", Europ Arch ORL, Suppl. 1995/I, 117-153) und der EP-A 0 456 166 bekannt.

Diese bekannten Verfahren basieren ausschließlich auf der Auswertung von Bildsequenzen des natürlichen Auges, die mit herkömmlichen Videokameras aufgezeichnet werden. Sie gestatten die Messung der horizontalen, vertikalen und torsionalen Augenposition mit einer Meßgenauigkeit von 0,1 Grad. Die jeweiligen Abtastraten entsprechen den Standard-Bildfrequenzen für Videosysteme, d.h. 25 bzw. 30 Hz. Dabei werden verschiedene Algorithmen zur Verfolgung des Pupillenmittelpunktes sowie natürlicher Kontrastmerkmale auf der Iris eingesetzt.

Wie bei den zweidimensionalen Systemen wird vorausgesetzt, dass der Pupillenmittelpunkt einen festen Punkt auf dem Augenbulbus darstellt, und somit als akkurater Indikator der horizontalen und vertikalen Position des Auges im Kopf dient. Hinzu kommt, dass der ermittelte Pupillenmittelpunkt auch als Referenzpunkt für die Definition der Irismerkmale herangezogen wird.

Die Ermittlung des Pupillenmittelpunktes kann auf verschiedenen Wegen erfolgen. Bei fast all diesen bekannten Methoden wird als erster Schritt das Augenbild binär quantisiert. Durch Vorgabe einer Luminanzschwelle kann die "schwarze Pupille" leicht segmentiert werden. Auf der Grundlage des so entstandenen binär quantisierten Bildes kann nun das Pupillenzentrum berechnet werden. Gängige Verfahren verwenden die Berechnung der Momente nullter Ordnung (sogenannte "centre-of-gravity-Methode") oder die geometrische Analyse der erkannten Randpunkte der "schwarzen Pupille".

Zusätzlich zu der Bestimmung der Pupillenkoordinaten wird nach CLARKE, A.H., TEIWES, W., SCHERER, H. ("Videooculography - an alternative method for the registration of eye movements in three dimensions", Proc. 5th European Conference on Eye Movements, Pavia 1989) die Drehung des Auges um den ermittelten Pupillenmittelpunkt (Augentorsion) anhand der sogenannten Polarkorrelation berechnet. Bei der Polarkorrelation wird von jedem Bild ein durch Polarkorrelation definiertes Kreissegment auf der Iris abgetastet. Die Lage des Segmentes wird für jedes Auge individuell so ausgewählt, dass eine sogenannte Irissignatur mit kontraststarken Merkmalen abgetastet werden. Eine anschließende Kreuzkorrelation der Irissignatur aus dem aktuellen gegenüber derer aus einem Referenzbild ermöglicht die Berechnung der relativen torsionalen Augenposition.

Bei dieser bekannten Methode ist es wichtig, die geometrischen Fehler zu korrigieren, die durch die orthogonale Projektion des kugelförmigen Auges auf der Sensorebene entstehen (HASLWATER T., MOORE S., "A theoretical analysis of three dimensional eye position measurement using polar cross-correlation", IEEE Trans ME 42, 11 1053-1061, 1995).

Es sind weiter eine Reihe ähnlicher Verfahren bekannt geworden (CLARKE, A.H. ("Neuere Aspekte des vestibuloolulären Reflexes", Europ Arch ORL, (Suppl. 1995/I)117-153), die zweidimensionale Korrelationsberechnungen bzw. Fast-Fourier-Transformationen einsetzen. Alle diese bekannten Verfahren arbeiten mit der Erkennung natürlicher Irissignaturen.

Das Bestimmen der horizontalen und vertikalen Augenkoordinaten aus dem Mittelpunkt der Pupille ist immer mit einem gewissen Fehler behaftet. Wie von WYATT, H.J. ("The form of the human pupil", Vision res. 35, 14, 2021-2036, 1995) beschrieben, ändert sich während der Dilatation bzw. Konstriktion auch die Form der Pupille und somit ändern sich auch die Koordinaten des Mittelpunktes, ohne dass eine Bewegung des Augapfels stattgefunden hätte.

Ein weiterer Nachteil ist dadurch bedingt, dass die Qualität der Messung der dritten Rotationskomponente, die Torsion um die Blickachse, sehr stark von der Ausprägung der natürlichen Irissignatur abhängig ist. Die Meßqualität wird daher vom "Rauschabstand" in der Irissignatur bestimmt.

Video-okulografische Verfahren arbeiten regelmäßig mit Infrarotbeleuchtung, die oft ein sehr kontrastarmes Bild der Irisstrukturen liefert, wodurch die Meßqualität weiter vermindert wird. Bei denjenigen Probanden mit schwach ausgeprägtem Irismuster mißlingt die zuverlässige Messung der torsionalen Komponente. Dieser Nachteil trifft auch für das aus der EP 0 456 166 B1 bekannte Verfahren der Extraktion eines natürlichen Irismerkmales zu.

Die bekannten Verfahren erfordern, bedingt durch die Grauwertverarbeitung der Irismerkmalen und den Korrelationsalgorithmus, einen sehr hohen Rechenaufwand, so dass eine Echtzeitmessung selbst bei der gängigen Bildfrequenz von 25 bzw. 30 Hz bisher nicht erreicht werden konnte.

Aus der DE-A 38 00 076 ist des weiteren ein Träger zur Aufnahme von optischen Markierungen oder optischen Abdeckungen oder Filtern oder sonstigen optisch relevanten Elementen zum Aufsetzen auf die menschliche Hornhaut bekannt. Auf der der Hornhaut zugewandten Seite sind an der Peripherie des Trägers kleine Spitzen angebracht, die sich in die Hornhaut oder in die Bindehaut des Auges einbohren, so dass der Träger relativ zum Auge bei Augenbewegungen fixiert bleibt. Der Träger dient zur Aufnahme von optischen Markierungen für Bewegungssensoren oder zur Ausblendung eines Teils des verwendeten Lichts.

Einerseits führt ein mehrmalige Aufsetzen des bekannten Trägers auf das Auge zu nicht vernachlässigbaren Deformationen bzw. Verletzungen der Horn- oder Bindehaut, andererseits ist das Markieren an ein- und derselben Stelle nicht mehr möglich, weil der Träger mit seinen Spitzen keinen Halt mehr findet.

Die Anwendung einer Markiertinte zum Auftragen auf die Bindehaut und die Hornhaut ist aus der JP-A 04-329 925 bekannt. Diese bekannte Markiertinte wird entweder mit einem Pinsel, Stift oder einer Düse aufgetragen und dient der Beobachtung der torsionalen Augenbewegung. Die nichtinvasive Tinte besteht aus einer Mixtur aus schwarzem Kohlenstoff und einem schnell trocknenden Bindemittel wie Polysacharid- oder Polyvinylalkohol. Sie klebt gleichermaßen auf der Haut und lässt sich nur mit von außen aufgebrachtem Wasser entfernen.

Eine Erfassung der horizontalen und vertikalen Bewegungskomponente ist nicht möglich.

Bei diesem Stand der Technik liegt der Erfindung die Aufgabe zugrunde, ein Verfahren und eine Anordnung der eingangs genannten Art derart zu verbessern, dass unter Wegfall der rechneraufwendigen Grauwertverarbeitung die Messung von horizontalen, vertikalen und torsionalen Bewegungskomponenten des Auges im Kopf mit hoher Auflösung und in Echtzeit ermöglicht wird.

Diese Aufgabe wird durch ein Verfahren der eingangs genannten Gattung mit den Merkmalen des Anspruches 1 und durch eine Anordnung mit den Merkmalen des Anspruches 11 gelöst.

Vorteilhafte Ausgestaltungen des Verfahrens und der Anordnung sind den Unteransprüchen entnehmbar.

Die Erfindung zeichnet sich vor allem dadurch aus, dass der Pupillenmittelpunkt bzw. der Bezugspunkt nur ein einziges Mal vor Beginn der Messung ermittelt werden muß. Es entfällt die rechnerintensive Graustufenverarbeitung des Augenbildes, so dass die Messung in Echtzeit möglich wird. Das Meßverfahren wird vereinfacht, ist kostengünstiger und zugleich genauer.

Die Markierungen aus der erfindungsgemäßen Tinktur werden nach der Messung problemlos durch die Tränenflüssigkeit des Probanden ausgewaschen. Es entfällt die Nachfolgeprozedur des Auswaschens mit Wasser aus dem Auge.

Weitere Vorteile und Einzelheiten ergeben sich aus der nachfolgenden Beschreibung unter Bezugnahme auf die beigefügten Zeichnungen.

Die Erfindung soll nachstehend an einem Ausführungsbeispiel näher erläutert werden.

Es zeigen:

Fig. 1          eine Darstellung eines Augapfels mit an diesem angreifenden Muskeln,

Fig. 2          eine Vorderansicht eines mit einer kreisförmigen Markierungen versehenen Auges,

Fig. 3          eine weitere Vorderansicht eines mit zwei kreisförmigen Markierungen versehenen Auges,

Fig. 4          eine schematische Darstellung der Lageveränderung der Markierung gemäß Fig. 1 im Falle einer Rotation des Auges um die Z-Achse des Auges,

Fig. 5          eine weitere schematische Darstellung der Lageveränderung der Markierung gemäß Fig. 1 im Falle einer Rotation des Auges um die Y-Achse des Auges,

Fig. 6          eine weitere schematische Darstellung der Lageveränderung der Markierung gemäß Fig. 1 im Falle einer Rotation des Auges um die torsionale Augenachse (Blickrichtung) und

Fig. 7 bis 9    in schematischer Darstellung die den Fig. 4 bis 6 entsprechenden Lageveränderungen von vier punktförmigen Markierungen.

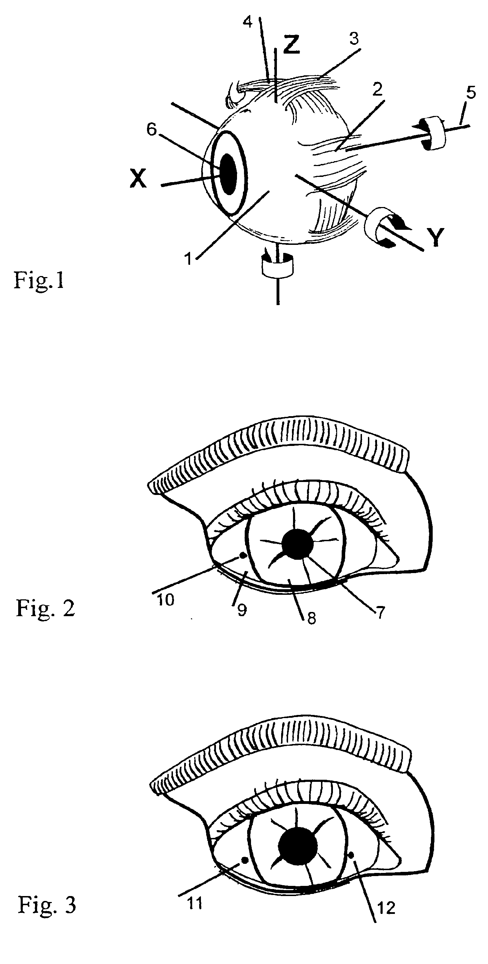

In Fig. 1 ist der Augapfel **1** mit den drei okulären Muskeln **2, 3, 4** dargestellt. Durch die Hebelwirkung dieser Muskeln wird das Auge um die Achsen **X,Y,Z** gedreht, wobei eine Drehung um die Z-Achse als horizontale, um die Y-Achse als vertikale und um die X-Achse als torsionale Augenbewegung bezeichnet wird. Dabei ist das orthogonale Koordinatensystem **5** so angeordnet, dass die X-Achse parallel zur Blickrichtung durch die Pupille **6** liegt.

In Fig. 2 ist schematisch ein mit einer Markierung **10** und in Fig. 3 ein mit zwei Markierungen **11, 12** versehenes Auge dargestellt. Dabei liegen die Markierungen außerhalb der Pupille **6** am Rand **7** zwischen Iris **8** und Sklera **9.**

Vor der Applikation der Markierungen wird das Auge mit einem herkömmlichen Lokalanästhetikum, beispielsweise Kerakain in Tropfenform, ähnlich wie vor einer Messung des okulären Druckes behandelt. Anschließend werden die Markierungen aus nichttoxischer Tinktur mit einem sterilen Stift entsprechend aufgebracht. Dabei werden die Markierungen stets nur auf der Bindehaut (Sklera) außerhalb der verletzbaren Kornea aufgebracht. Die aufgebrachten Markierungen werden aus dem Auge nach etwa einer Stunde auf natürliche Weise durch die Tränenflüssigkeit ausgewaschen. Als besonders vorteilhaft erweist sich die Verwendung einer oder mehrerer kreisförmiger Markierungen. Bei der Durchführung des Verfahrens wird in bekannter Weise eine Bildsequenz des Auges mit Hilfe eines Videosensors oder anderweitigen optoelektronischen Matrix erfaßt und mit digitalen Bildverarbeitungstechniken verarbeitet. Unter unsichtbarer Infrarotbeleuchtung, beispielsweise LED's mit einer Wellenlänge von 880 bzw. 930 nm, setzen sich die aufgebrachten Markierungen deutlich von den übrigen Teilen des Bildes ab, so dass eine Schwellwerttrennung des Luminanzsignals möglich wird. Auf diese Weise wird jedes Bild der Sequenz binarisiert und die Koordinaten der zwei (oder mehreren) diskriminierten Objekte mit bekannten Algorithmen berechnet. Da die Form und die Anordnung der aufgebrachten Markierungen vorgegeben ist, eignen sich hierfür die Berechnung des nullten und ersten Moments der Markierungsform sowie die Anwendung eines "template-matching"-Verfahrens (vgl. CLARKE A.A. u.a. in Elsevier).

Mit diesem Ansatz entfällt das komplexe Graustufenberechnungsverfahren (FFT, Korrelation), so dass eine Echtzeiterfassung und darüber hinaus eine Erhöhung der Messung (d.h. höhere Bild-Abtastfrequenz mit entsprechend schnellen optoelektronischen Bildsensoren) erreicht wird.

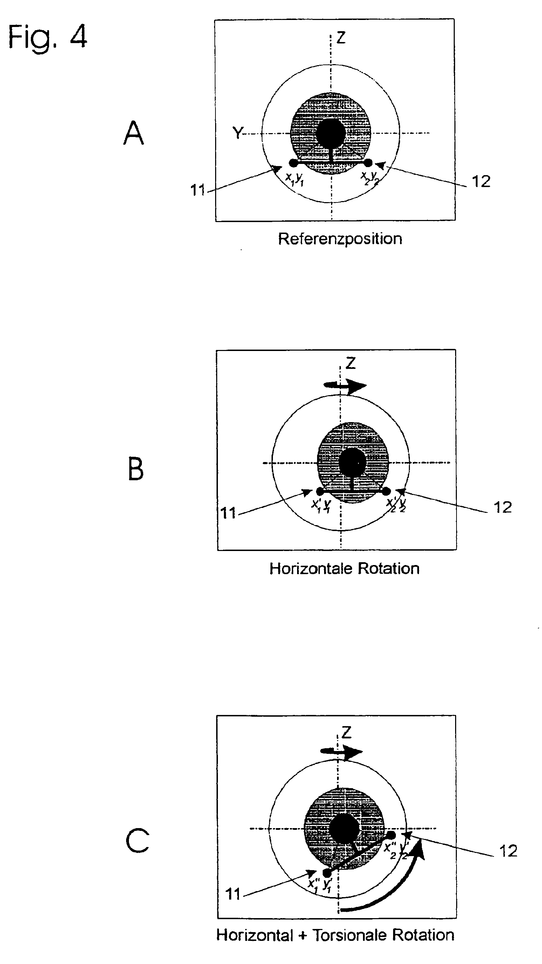

Fig. 4 A (oben) zeigt die Lage der Markierung **11** und **12** in der Referenzposition und Fig. 4B (Mitte) die Lage der Markierungen **11** und **12** nach einer Rotation um die Z-Achse (horizontale Rotation). In Fig. 4C ist die Lage der Markierungen 11 und 12 nach einer obliquen Rotation um die Z-Achse und und dann um die X-achse (torsional) dargestellt.

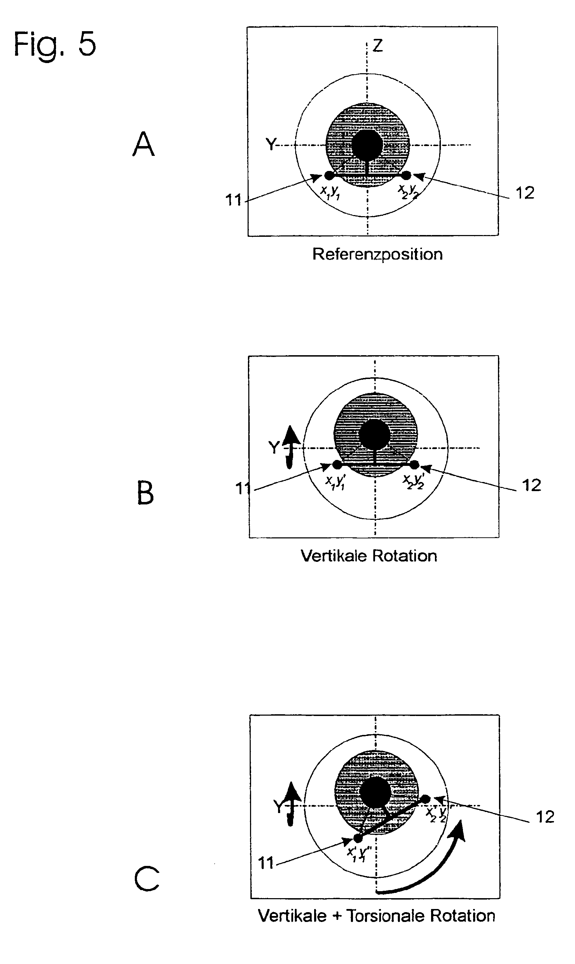

Fig. 5 A und 5 B zeigt die Lagen der Markierungen **11** und **12** vor und nach einer Rotation um die Y-Achse (vertikale Rotation). Fig. 5 C stellt die Lage der Markierungen 11 und 12 nach einer obliquen Drehung um die Y-Achse, gefolgt von einer Drehung um die X-Achse dar.

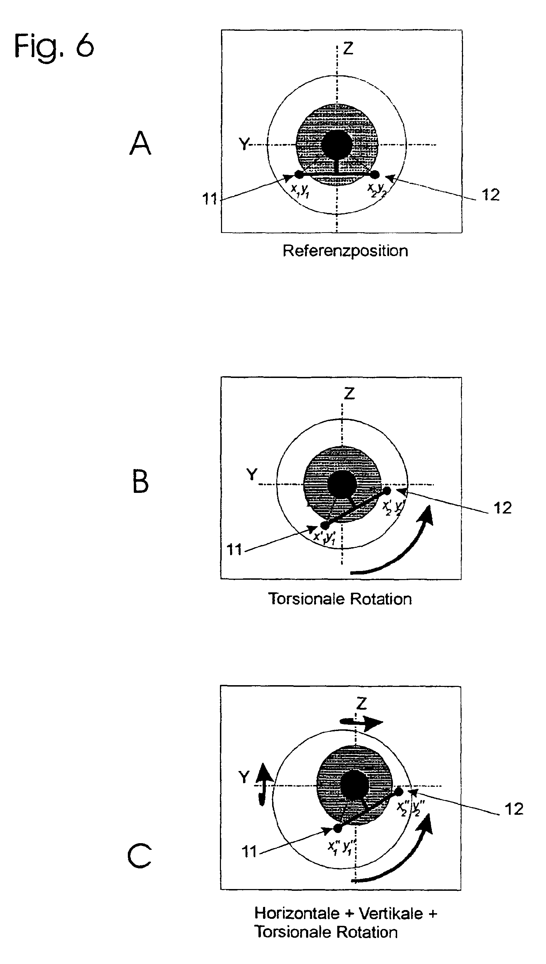

In Fig. 6 A und Fig. 6 B sind die Lagen der Markierung **11** und **12** vor (oben) und nach (Mitte) einer Rotation um die torsionale Augenachse **X** illustriert.

Fig. 6 C zeigt die Lage der Markierungen 11 und 12 nach einer obliquen Drehung um die Z-Achse, um die Y-Achse und um die X-Achse.

Entsprechend der Bildsequenz entsteht eine zeitliche Folge von Koordinatendaten, die die Berechnung der drei Rotationskomponenten der Augenbewegung auf einfache Weise erlaubt.

Ähnlich dem von HASLWATER T. und MOORE S. ("A theoretical analysis of three dimensional eye position measurement using polar cross-correlation", IEEE Trans ME 42, 11 1053-1061, 1995) beschriebenen Verfahrens kann hier auch eine Korrektur des Projektionsfehlers vorgenommen werden.

Ein wichtiger Aspekt besteht darin, die Zweideutigkeit der Anordnung von jeweils zwei aufgebrachten Markierungen für die Bestimmung der Augenposition zu nutzen.

Aus Fig. 4 bis 6 ist zu ersehen, dass sobald eine Änderung der torsionalen Position eintritt, es fälschlicherweise zu einer horizontalen bzw. vertikalen Verschiebung kommt. Durch die Einbeziehung der geometrischen Anordnung' des Pupillenmittelpunktes zu den beiden Markierungen läßt sich über die Abbildung des Auges eine Fläche aufspannen, die die eindeutige Berechnung der horizontalen, vertikalen und torsionalen Position erlaubt. Hierfür muß der Pupillenmittelpunkt nur einmal bei der Meßvorbereitung durchgeführt werden, am besten mit dem Auge in der "Geradeaus"-Referenzposition. Da angenommen wird, dass das Auge ein fester Körper ist und somit die Anordnung der Markierungen und der Pupillenmittelpunkt ortsfest ist, kann danach dieser dritte Punkt für jedes Bild von den Koordinaten der beiden Markierungen, und somit die dreidimensionale Position des Auges numerisch ermittelt werden.

Die horizontale und vertikale Koordinaten der Augenposition sind durch die ermittelten Koordinaten des dritten Punktes des aufgespannten Dreiecks, d.h. des rekonstruierten Pupillenmittelpunktes, gegeben.

Die Drehung des Auges um Blickachse wird durch Ermittlung der Lage der Verbindungslinien zwischen den Markierungen mit deren Ausgangs- bzw. Referenzlage

$$\psi_i = \psi_{akt} - \psi_{null}$$

bestimmt.

Natürlich ist es auch möglich, durch zusätzliche Markierungen ohne Bezug auf den Pupillenmittelpunkt eine entsprechende Fläche zur Ermittlung der Augenposition auf dem Augenbild aufzuspannen.

Im letzten Schritt werden aus der Folge der Objektkoordinaten aus jedem Bild die drei Rotationskomponenten der Augenbewegung in Grad-Einheiten bestimmt.

Dies geschieht beispielsweise auf folgende Weise:

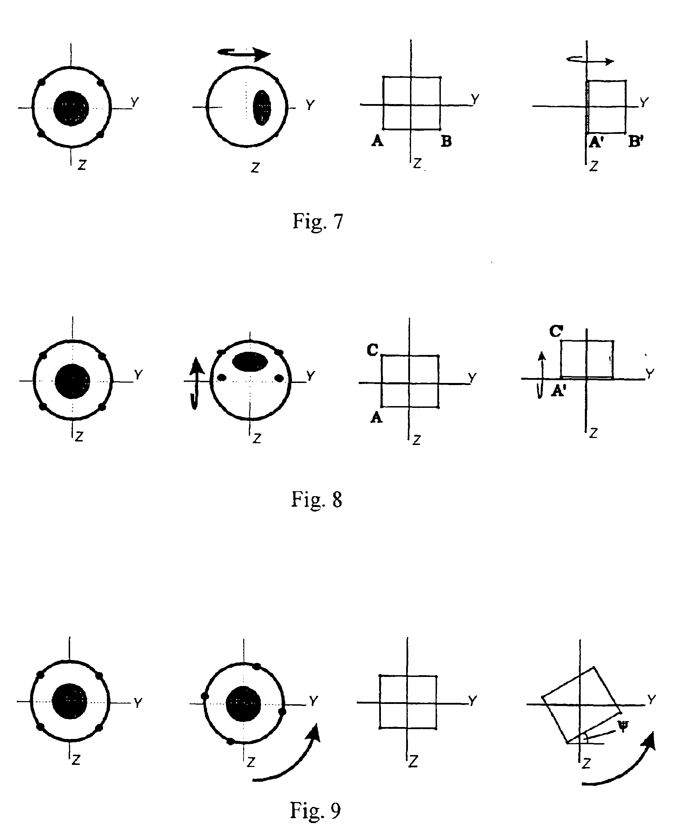

Mit Hilfe einer Blickwinkelkalibrierung wird der effektive Radius des Augapfels bestimmt. Dabei betrachtet der Proband mit dem zu messenden Auge eine Tafel mit einem Achsenkreuz, auf dem punktförmige Lichtquellen in bestimmten Winkelabständen montiert sind (siehe Fig. 9).

Da der Abstand zwischen Auge und Tafel im Vergleich zum Augenradius groß ist, lassen sich die Blickwinkel zwischen dem mittleren Punkt und den exzentrischen Punkten auf den Achsen durch $\Phi = \arctan(y/a)$ bzw. $\theta = \arctan(x/a)$ berechnen. Die Augenbewegungen werden gemessen, während der Proband abwechselnd die Lichtpunkte fixiert.

Die Berechnung des Augenradius $\Gamma_{auge}$ erfolgt nach folgender Gleichung

$$\begin{bmatrix} X_i \\ y_i \end{bmatrix} = \begin{bmatrix} \Gamma_{auge}\ \sin\theta_i \cos\phi_i \\ \Gamma_{auge}\ \sin\phi_i \end{bmatrix}$$

für jeden der fixierten Punkte, dessen Winkel ungleich null ist. Diese Gleichung ergibt sich aus der klassischen Lehre der Rotationstransformation (siehe HASLWANTER T, MOORE S., "A theoretical analysis of three dimensional eye position measurement using polar cross-correlation", IEEE Trans BME 42, 11, 1053-1061).

Wenn der Drehradius des Augapfels bekannt ist, kann die horizontale und vertikale Koordinate des Auges für jeden Abtastpunkt anhand der Gleichungen

$$\theta = \arcsin((x_i - x_{i+1})/\Gamma_{auge}); \quad \Phi = \arcsin((y_i - y_{i+1})/\Gamma_{auge})$$

bestimmt werden.

In den Fig. 7 bis 9 ist eine Anordnung dargestellt, bei der mit vier punktförmigen Markierungen gearbeitet wird. Diese Lösung erlaubt eine Bestimmung der Augenbewegung ohne Ermittlung des Augenradius bzw. ohne Kalibrierungsprozedur. Die Fig. 7 zeigt die Lage der Markierungen bei einer Drehung um die horizontale, Fig. 8 eine Drehung um die vertikale und Fig. 9 eine Drehung um die torsionale Achse mit den entsprechenden Projektionsverzerrungen.

Wegen der kugelförmigen Form des Augapfels führt eine Drehung um die horizontale bzw. vertikale Achse zu einer Verzerrung in der Projektion der Markierungen bzw. ihrer Anordnung auf der Abbildungsebene des Bildsensors. Die Drehung um die Z- und Y-Achsen kann durch trigonometrische Berechnung bestimmt werden. Der Drehwinkel, der durch eine Verkürzung bzw. Verlängerung der orthographischen Projektion einer horizontalen bzw. vertikalen Linie (Markierung oder Abstand zwischen zwei Markierungen) abgebildet wird, entspricht

$$\theta = \arccos(A'B'/AB) \quad bzw. \quad \Phi = \arccos(A'C'/AC).$$

Bei beiden Ansätzen wird die Drehung des Auges um die Blickachse durch Bestimmung der Lage der Markierung dargestellt bzw. der Verbindungslinien zwischen den Markierungen mit deren Referenzlagen ermittelt.

Die Tinktur zur künstlichen Markierung der Bindehaut des Auges für mehrdimensionale Augenbewegungen besteht aus einem gesundheitsverträglichen, auf dem Epithel der Bindehaut temporär anhaftenden Farbstoff auf Eisenoxidbasis und Wasser, wobei die Tinktur mehr als 80 Gew.-% Eisen enthält. Vorteilhafterweise ist in der Tinktur $Fe_2O_4$ enthalten. Künstliche Markierungen mit dieser Tinktur sind ohne zusätzliche Anwendung von Wasser durch die Tränenflüssigkeit auswaschbar.

Aufstellung der verwendeten Bezugszeichen

| | |
|---|---|
| Augapfel | 1 |
| okuläre Muskel | 2, 3, 4 |
| orthogonales Koordinatensystem | 5 |
| Pupille | 6 |
| Rand der Pupille | 7 |
| Iris | 8 |
| Sklera | 9 |
| Markierung | 10 |
| weitere Markierungen | 11, 12 |
| torsionale Achse (Blickachse) | X |
| horizontale Achse | Z |
| vertikale Achse | Y |
| Winkelstellung um das Drehzentrum | $\theta$ |
| Winkeldrehung um das Drehzentrum | $\phi$ |
| Winkeldrehung um die Blickachse | $\Psi$ |

**Patentansprüche**

1. Verfahren zum Erfassen von mehrdimensionalen Augerbewegungen, insbesondere horizontalen, vertikalen und torsionalen Augenbewegungen, bei dem das Auge mit diffusem Infrarotlicht beleuchtet wird und durch einen optoelektronischen Sensor Bildsequenzen des Auges erfasst werden, die einer dedizierten Rechenarchitektur zur Verarbeitung zugeführt werden, die die Koordinaten der Augenbewegung bestimmt und wahlweise die Bildsequenzen mit den ermittelten Koordinaten auf einem Massenspeicher aufzeichnet und auf einem Monitor anzeigt, umfassend folgende Schritte:

   a) Aufbringen von mindestens zwei Markierungen in einer vorgegebenen Geometrie auf die Bindehaut des Auges ausserhalb der Kornea zur Herstellung einer eindeutigen Kontrast-Signatur unter Infrarotbeleuchtung, wobei die Markierungen mittels einer nichttoxischen Tinktur oder mittels Laserstrahlen aufgebracht werden;

b) einmaliges Bestimmen der Koordinaten eines rotationsneutralen Bezugspunktes (Pupillenmittelpunkt beim Geradeausblick) als Referenzposition vor Beginn der Messung;

c) Erfassen, Digitalisieren und Speichern der Bildsequenzen mit dem optoelektronischen Sensor,

d) Binarisieren und Segmentieren eines jeden Augenbildes durch Schwellwerttrennung nach der künstlichen Kontrast-Signatur und Bestimmen der Koordinaten der Markierungen gemäss Schritt b);

e) Zuordnen der Koordinaten des rotationsneutralen Bezugspunktes zu den Koordinaten der Markierungen und Aufspannen einer Flache über jedem segmentierten Augenbild,

f) Bestimmen der Schwerpunkte aus den voneinander beabstandeten Markierungen nach Schritt a) und Ermittlung der Abweichung von den Koordinaten des rekonstruierten Bezugspunktes nach Schritt b) als Mass für die horizontale, vertikale und torsionale Position des Auges.

2. Verfahren nach Anspruch 1, **dadurch gekennzeichnet, dass** die Koordinaten von zwei oder mehreren künstlich Markierungen ermittelt werden, so dass die Erfassung der Augenbewegung unabhängig von der Verfolgung natürlicher Merkmale, beispielsweise der Pupille, durchführbar ist.

3. Verfahren nach Anspruch 1 oder 2, **dadurch gekennzeichnet, dass** als Anordnung der Markierungen eine einfache geometrische Form, vorzugsweise eine Dreiecksform oder Rechtecksform, gewählt wird, deren Eckpunkte die Koordinatenpaare bilden.

4. Verfahren nach einem der Ansprüche 1 bis 3, **dadurch gekennzeichnet, dass** die Markierungen im Bereich desUbergangs von der Iris zur Sklera aufgebracht werden.

5. Verfahren einem der Ansprüche 1 bis 4, **dadurch gekennzeichnet, dass** die Markierungen selbst einfache geometrische Formen, vorzugsweise Kreise, bilden.

6. Verfahren einem der Ansprüche 1 bis 5, **dadurch gekennzeichnet, dass** die Markierungen mittels eines mit der Tinktur getränkten Faserstifts auf der Bundehaut positioniert werden.

7. Verfahren einem der Ansprüche 1 bis 6, **dadurch gekennzeichnet, dass** als Markierungs-Tinktur eine lebensmittelverträgliche farbstoffhaltige Lösung verwendet wird, die unter Infrarotbeleuchtung detektierbar ist.

8. Verfahren nach Anspruch 7, **dadurch gekennzeichnet, dass** als Farbstoff Eisenoxide, vorzugsweise $Fe_3O_4$, verwendet werden.

9. Verfahren einem der Ansprüche 1 bis 8, **dadurch gekennzeichnet, dass** die Tinktur aus einem gesundheitsverträglichen, auf dem Epithel der Bindehaut temporär anhaftenden Farbstoff auf Eisenoxidbasis und Wasser besteht, wobei die Tinktur mehr als 80 Gew.-% Eisen enthält

10. Verfahren nach Anspruch 1 bis 9, **dadurch gekennzeichnet, dass** als optoelektronische Sensoren, vorzugsweise CCD-oder CMOS-Bildsensoren, verwendet werden.

11. Anordnung zum Erfassen mehrdimensionaler Augenbewegungen, mit einer diffusen IR-Lichtquelle zur Beleuchtung des Auges, einem optoelektronischen Sensor zur Erfassung von Bildsequenzen des Auges, und einer dedizierten Rechenarchitektur zur Bildverarbeitung für die Bestimmung der Augenbewegungen mittels künstlich auf der Bindehaut in einer vorgegebenen Geometrie aufgebrachten Markierungen, **dadurch gekennzeichnet, dass** die dedizierte Rechnerarchitektur zur Durchführung der Verfahrensschritte (b) bis (f) gemäß Patentanspruch 1 ausgestaltet ist.

**Claims**

1. Method for detecting multi-dimensional eye movements, in particular horizontal, vertical and torsional eye movements, wherein the eye is illuminated with diffuse infrared light, and by means of an optoelectronic sensor image sequences of the eye are detected which are supplied to a dedicated computational architecture for processing which determines the coordinates of the eye movement and selectively records the image sequences with the determined coordinates on a mass storage device and displays them on a monitor, comprising the following steps:

a) application of at least two markings according to a preset geometry to the conjunctiva of the eye outside the cornea in order to produce a clear contrast signature under infrared illumination, wherein the markings are applied using a non-toxic tincture or by laser radiation;

b) one-off determination of the co-ordinates of a rotationally neutral reference point (centre of the pupil when looking straight ahead) as a reference position before measurement begins;

c) detection, digitisation and storage of the image sequences using the optoelectronic sensor,

d) conversion into binary form and segmentation of each eye image by threshold value separation according to the artificial contrast signature and determination of the co-ordinates of the markings in accordance with step b);

e) assignment of the co-ordinates of the rotationally neutral reference point to the co-ordinates of the markings and extending a surface over each segmented eye image,

f) determination of the centroids of the mutually spaced markings in accordance with step a) and detection of the deviation from the co-ordinates of the reconstructed reference point in accordance with step b) as a measurement for the horizontal, vertical and torsional position of the eye.

2. Method as claimed in claim 1, **characterised in that** the coordinates of two or more artificial markings are determined so that the detection of the eye movement can be carried out independently of the tracing of natural features such as the pupil.

3. Method as claimed in claim 1 or 2, **characterised in that** a simple geometrical shape, preferably a triangular shape or rectangular shape, the corners of which form the coordinate pairs, is selected for positioning of the markings.

4. Method as claimed in any one of claims 1 to 3, **characterised in that** the markings are applied in the region of the transition from the iris to the sclera.

5. Method as claimed in any one of claims 1 to 4, **characterised in that** the markings themselves form simple geometric shapes, preferably circles.

6. Method as claimed in any one of claims 1 to 5, **characterised in that** the markings are positioned on the conjunctiva by means of a fibre pen soaked in the tincture.

7. Method as claimed in any one of claims 1 to 6, **characterised in that** a food-grade dye-containing solution which can be detected under infrared illumination is used as the marking tincture.

8. Method as claimed in claim 7, **characterised in that** iron oxides, preferably $Fe_3O_4$, are used as the dye.

9. Method as claimed in any one of claims 1 to 8, **characterised in that** the tincture consists of an iron oxide-based dye, which is not harmful to health and which temporarily adheres to the epithelium of the conjunctiva, and water, wherein the tincture contains more than 80% by weight of iron.

10. Method as claimed in claims 1 to 9, **characterised in that** CCD or CMOS image sensors are preferably used as the optoelectronic sensors.

11. Assembly for detecting multi-dimensional eye movements, having a diffuse IR light source to illuminate the eye, an optoelectronic sensor for detecting image sequences of the eye, and a dedicated computational architecture for image processing to determine the eye movements by means of markings artificially applied to the conjunctiva according to a preset geometry, **characterised in that** the dedicated computer architecture is constructed to carry out the method steps (b) to (f) in accordance with claim 1.

**Revendications**

1. Procédé pour détecter des mouvements oculaires multidimensionnels, notamment des mouvements oculaires horizontaux, verticaux et torsionnels, dans lequel l'oeil est éclairé avec de la lumière infrarouge diffuse et dans lequel un capteur optoélectronique détecte des séquences d'images de l'oeil, lesquelles sont dirigées pour traitement vers une architecture de calcul dédiée qui détermine les coordonnées des mouvements oculaires et enregistre au choix les séquences d'images avec les coordonnées déterminées sur une mémoire de masse et les affiche sur un moniteur, comprenant les étapes suivantes :

a) application d'au moins deux marquages, dans une géométrie prédéfinie sur la conjonctive de l'oeil, à l'extérieur de la cornée, pour la création d'une signature par contraste unique sous éclairage par infrarouge, les marquages étant appliqués au moyen d'une teinture non toxique ou au moyen de rayons laser ;

b) détermination unique des coordonnées d'un point de référence neutre en rotation (point médian de la pupille, lors d'un regard droit vers l'avant) en tant que position de référence, avant le début de la mesure ;

c) détection, numérisation et mémorisation des séquences d'images avec le capteur optoélectronique,

d) binarisation et segmentation de chaque image de l'oeil par séparation des valeurs seuils selon la signature par contraste artificielle et définition des coordonnées du marquage selon l'étape b) ;

e) association des coordonnées du point de référence neutre en rotation aux coordonnées du marquage et montage d'une surface par-dessus chaque image d'oeil segmentée,

f) définition des centres de gravité à partir des marquages écartés les uns des autres selon l'étape a) et détermination de la différence avec les coordonnées du point de référence reconstruit selon l'étape b), en tant que mesure pour la position horizontale, verticale et torsionnelle de l'oeil.

2. Procédé selon la revendication 1, **caractérisé en ce que** les coordonnées de deux ou de plusieurs marquages artificiels sont déterminées, pour que la détection du mouvement oculaire soit réalisable indépendamment de la poursuite de caractéristiques naturelles, par exemple de la pupille.

3. Procédé selon la revendication 1 ou 2, **caractérisé en ce que**, en tant que structure du marquage, on choisit une forme géométrique simple, de préférence une forme triangulaire ou une forme rectangulaire, dont les points angulaires forment les paires de coordonnées.

4. Procédé selon l'une quelconque des revendications 1 à 3, **caractérisé en ce que** les marquages sont appliqués dans la zone du passage de l'iris à la sclérotique.

5. Procédé selon l'une quelconque des revendications 1 à 4, **caractérisé en ce que** les marquages proprement dits représentent des formes géométriques, de préférence des cercles.

6. Procédé selon l'une quelconque des revendications 1 à 5, **caractérisé en ce que** les marquages sont positionnés au moyen d'un crayon feutre sur la conjonctive.

7. Procédé selon l'une quelconque des revendications 1 à 6, **caractérisé en ce que**, en tant que teinture de marquage, on utilise une solution contenant du colorant alimentaire, qui est détectable sous éclairage par infrarouge.

8. Procédé selon la revendication 7, **caractérisé, en ce qu'**on utilise en tant que colorant des oxydes de fer, de préférence du $Fe_3O_4$.

9. Procédé selon l'une quelconque des revendications 1 à 8, **caractérisé en ce que** la teinture consiste dans un colorant compatible avec la santé, adhérant temporairement sur l'épithélium de la conjonctive, sur base d'oxyde de fer et d'eau, la teinture contenant plus de 80 % en poids de fer.

10. Procédé selon la revendication 1 à 9, **caractérisé en ce qu'**on utilise en tant que capteurs optoélectronique de préférence des capteurs d'images CCD ou CMOS.

11. Structure pour détecter des mouvements oculaires pluridimensionnels, comprenant une source d'image IR diffuse pour éclairer l'oeil, un capteur optoélectronique pour détecter des séquences d'images de l'oeil, et une architecture de calcul dédiée pour le traitement d'images pour définir les mouvements oculaires au moyen de marquages appliqués artificiellement sur la conjonctive selon une géométrie prédéfinie, **caractérisée en ce que** l'architecture de calcul dédiée est conçue pour la réalisation des étapes de procédé (b) à (f) selon la revendication 1.

Fig.1

Fig. 2

Fig. 3

Fig. 4

A

Referenzposition

B

Horizontale Rotation

C

Horizontal + Torsionale Rotation

Fig. 5

A

Referenzposition

B

Vertikale Rotation

C

Vertikale + Torsionale Rotation

# Fig. 6

A

Referenzposition

B

Torsionale Rotation

C

Horizontale + Vertikale +
Torsionale Rotation

Fig. 7

Fig. 8

Fig. 9

**EP 1 241 976 B1**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 0456166 A
- EP 0456166 B1
- DE 3800076 A
- JP 4329925 A

**In der Beschreibung aufgeführte Nicht-Patentliteratur**

- Videooculography - an alternative method for measurement of three-dimensional eye movements. **CLARKE A. H. ; TEIWES W ; SCHERER, H.** Oculomotor Control and Cognitive Processes. Elsevier, 1991, 431-443
- **WYATT, H.J.** The form of the human pupil. *Vision Res.,* 1995, vol. 35 (14), 2021-2036
- **CLARKE A.H. ; TEIWES W. ; SCHERER H.** Videooculography - an alternativ method for the registration of eye movements in three dimensions. *5th European Conference on Eye Movements, Pavia,* 1993
- Image processing techniques for the measurement of eye movement. **CLARKE A.H.** Eye Movements in Reading. Elsevier, 1991, 21-38
- **CLARKE A.H.** Neuere Aspekte des vestibulookulären Reflexes. *Europ Arch ORL, Suppl.,* 1995, 117-153
- **CLARKE, A.H ; TEIWES, W. ; SCHERER, H.** Videooculography - an alternative method for the registration of eye movements in three dimensions. *Proc. 5th European Conference on Eye Movements, Pavia,* 1989
- **HASLWATER T. ; MOORE S.** A theoretical analysis of three dimensional eye position measurement using polar cross-correlation. *IEEE Trans ME,* 1995, vol. 42 (11), 1053-1061
- **CLARKE, A.H.** Neuere Aspekte des vestibuloolulären Reflexes. *Europ Arch ORL,* 1995, 117-153
- **VON WYATT, H.J.** The form of the human pupil. *Vision res,* 1995, vol. 35 (14), 2021-2036
- template-matching. **CLARKE A.A.** Verfahrens. Elsevier
- **MOORE S.** A theoretical analysis of three dimensional eye position measurement using polar cross-correlation. *IEEE Trans ME,* 1995, vol. 42 (11), 1053-1061
- **HASLWANTER T ; MOORE S.** A theoretical analysis of three dimensional eye position measurement using polar cross-correlation. *IEEE Trans BME,* vol. 42 (11), 1053-1061